# EUROPEAN PATENT SPECIFICATION

(11) **EP 4 051 328 B1**
(45) Date of publication and mention of the grant of the patent: **11.03.2026**
(21) Application number: 20807984.8
(22) Date of filing: 27.10.2020
(51) Int. Cl.: A61L 29/08, A61L 29/14, A61L 29/16

(54) **URINARY CATHETERS AND METHODS FOR PREVENTING BACTERIAL INFECTIONS**
HARNKATHETER UND VERFAHREN ZUM VORBEUGEN BAKTERIELLER INFEKTIONEN
CATHÉTERS URINAIRES ET PROCÉDÉS POUR PRÉVENIR DES INFECTIONS BACTÉRIENNES

(30) Priority: 28.10.2019 US 201962926891 P
(43) Date of publication of application: 07.09.2022
(73) Proprietor: Hollister Incorporated, Libertyville, IL 60048 (US)
(72) Inventor: SILEIKA, Tadas S., Libertyville, IL 60048 (US); DEAN, Nicole L., Libertyville, IL 60048 (US); PETTIGREW, Jacqueline MJ, Libertyville, IL 60048 (US)
(74) Representative: FRKelly
(86) International application number: PCT/US2020/057488
(87) International publication number: WO 2021/086831

(56) References cited:
- US-A1- 2010 198 195
- CHAN MICHELLE ET AL: "Inhibition of bacterial motility and spreading via release of cranberry derived materials from silicone substrates", COLLOIDS AND SURFACES B: BIOINTERFACES, ELSEVIER AMSTERDAM, NL, vol. 110, 29 April 2013 (2013-04-29), pages 275 - 280, XP028573101, ISSN: 0927-7765, DOI: 10.1016/J.COLSURFB.2013.03.047

## Description

The present application claims the benefit of and prior to U.S. Patent Application No. 62/926,891, filed October 28, 2019.

### DESCRIPTION

### TECHNICAL FIELD

The present disclosure generally relates to urinary catheters and methods for preventing bacterial infections, and more particularly, to catheters and methods for preventing urinary tract infections. The present disclosure also relates to urinary catheters and methods that inhibit the motility of bacteria within the urethra.

### BACKGROUND

It is desirable for urinary catheters to have a lubricated or lubricious outer surface to increase comfort of the patient during insertion into and/or removal from the body. One method for rendering the surface of a urinary catheter lubricious is to coat at least the insertable portion of the catheter with a lubricious hydrophilic coating. Another method is to apply a lubricant to the urinary catheter. Lubricity of the catheter minimizes soft tissue damage and reduces overall discomfort during use of the medical device.

Although catheters are typically prepared in sterile environments and are provided with safe handling instructions, catheter users are at risk of contracting a urinary tract infection due to several different factors. For example, the catheter may become contaminated during use and introduce bacteria into the urethra. US 2010/198195 A1 discloses urethral catheters that minimize the introduction of pathogenic organisms. The catheter also may carry bacteria along the urinary tract.
Additionally, because urine is voided from the bladder through the catheter, urine does not flow directly through the urethra. Thus, urine is no longer a factor in flushing out the urethra or wetting of the urethral tissue.

Therefore, there is a need for catheters, which reduce the risk of urinary tract infections.

### SUMMARY

There are several aspects of the present subject matter, which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a urinary catheter product for insertion into the urethra, wherein the urethra contains urethral mucus having a viscosity. The catheter product includes, a package (12) containing: the catheter (14) comprising a catheter shaft (16) having a hydrophilic coating on the catheter shaft (16); a hydration medium (18) for hydrating the hydrophilic coating; and a compound contained in the hydration medium (18), the compound for retaining and/or increasing the viscosity of the urethral mucus and, wherein the compound comprises a desiccant that removes moisture from the urethral mucus.

### BRIEF DESCRIPTION OF THE DRAWINGS

Fig. 1 is a plan view of one embodiment of the present disclosure; and
Fig. 2 is a plan view of one embodiment of a catheter of the present disclosure.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

For catheter users, whether in-dwelling or intermittent, urine is no longer a factor that participates in regular flushing or wetting of the urethra. Because the users are unable to void directly through the urethra, the urethra no longer experiences the continuous flushing and re-wetting of the tissues that normally occurs during active voluntary urination. When the mechanism fails, the risk of urinary tract infections increases.

Urinary tract infections can be a result of bacteria swarming and traveling up through the urethra towards the bladder. Traveling of bacteria up the urethra is typically not a concern for those who can perform voluntary urination, because normal voiding tends to wash out and eliminate the bacteria from urethra, thereby prohibiting it from traveling toward the bladder and causing infection.

There are certain species of bacteria that are considered good "swimmers," which swarm and move when they receive certain signals from the surrounding environment. These bacteria are capable of movement even on solid surfaces through excretion of their own extracellular fluids. These motile bacteria also are known to become more pathogenic and increase their motility organelles, such as flagella (a process called hyperflagellation), when sensing certain signals, such as decreased viscosity in the fluids and mucus in their surrounding environment.

In a non-voiding patient, the urethral environment will be fairly viscous, due to the presence of mucosal secretions. When a hydrophilic catheter or a catheter lubricated with a water-based gel is introduced into the urethra, such introduction will likely deposit additional moisture, diluting the secretions, thus decreasing the overall viscosity of the urethral mucus and the surrounding urethral environment. This decrease in the urethral mucus viscosity provides a signal that activates the motility of the bacteria.

The catheter products disclosed herein inhibit bacteria mobility and/or mitigate the risk of bacteria swarming and traveling proximally up the urethra toward the bladder. The catheter products include a compound for retaining and/or increasing the viscosity of the urethral mucus (which includes the fluids and other substances within the urethra). As discussed above, when the viscosity of the urethral mucus is decreased, such decrease can provide a signal to the bacteria that activates motility of bacteria. When the viscosity of the urethral mucus is retained and/or increased, there is no decreased viscosity signal and the motility of bacteria is not activated. Thus, the bacteria remain static and are not activated to swarm and travel up the urethra toward the bladder.

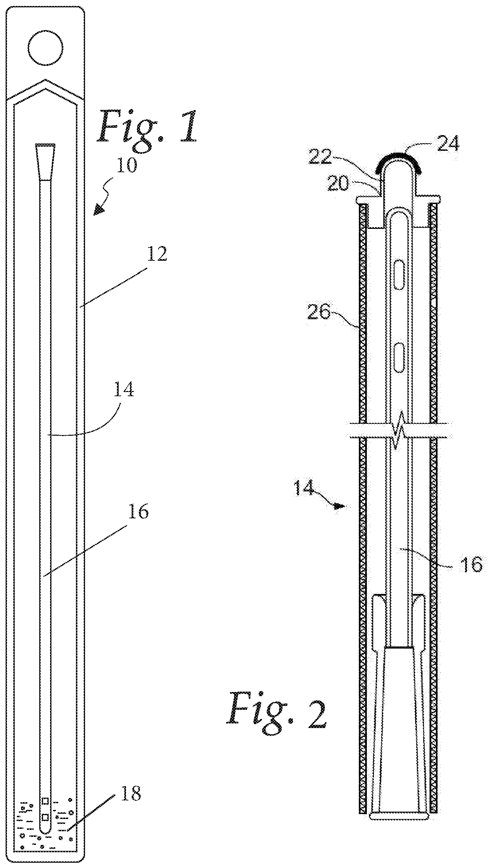

The compound for retaining and/or increasing the viscosity of the urethral mucous may be included in any component of a catheter product. For example, referring to Fig. 1, a catheter product 10 includes a package 12 including a catheter 14 having a catheter shaft 16. The outer surface of the catheter shaft 16 may be hydrophilic. For example, the catheter 16 may be coated with a hydrophilic coating. The hydrophilic surface of the catheter shaft 16 becomes lubricious when wetted with a hydration medium 18 (water or saline). The compound for retaining and/or increasing the viscosity of the urethral mucous is contained in the hydration medium and may be contained in the hydrophilic material (e.g., the hydrophilic coating). When the catheter product includes a lubricating gel, the compound may be included in the lubricating gel.

Referring to Fig. 2, when the catheter product includes an introducer aid 20 that may be inserted into the urethral opening and which the catheter is inserted through, the introducer aid 20 or the tip 22 thereof may include the compound 24. When the catheter shaft 16 is inserted through the introducer aid 20, the retaining and/or viscosity increasing compound may be disposed onto the catheter shaft 16 wherein the catheter distributes the compound along the urethra. Additionally, a sleeve 26 may be attached to the introducer aid, wherein the sleeve surrounds the catheter.

In one embodiment, the catheter includes a compound that dries out the urethral environment and/or the urethral mucus, by removing water from the urethral environment. The removal of water from the urethral environment maintains and/or increases the viscosity of the environment. In one example, the compound removes any residual moisture following catheterization. The residual moisture could be associated with either the presence of mucosal secretions and/or the water left behind from the insertion of hydrophilic catheter or water based gel-lubricant catheter.

The compound included in the catheter product to assist in drying out the urethral environment may be a desiccant that removes moisture from the urethral mucus. The desiccant may include one or more of alcohol, volatile solvents, antihistamines, and loperamide hydrochloride. As mentioned above, the desiccant is included in the hydration medium and may be included in a hydrophilic coating, gel-lubricant or insertion tip of the catheter.

In another embodiment, not according to the invention, the compound that maintains or increases the viscosity of the urethral environment may be a thickening agent that the catheter deposits into the urethral environment. The thickening agent may offset any moisture that is placed into the urethral environment by catheterization and/or the thickening agents may increase or maintain the original viscosity of the urethral environment. When the viscosity of the urethral mucus is retained and/or increased, there is no decreased viscosity signal and the motility of bacteria is not activated. Thus, the bacteria remain static and are not activated to swarm and travel up the urethra toward the bladder.

The thickening agent could be for example, gelling agents, such as agar or gelatin, or physical hydrogels, such as that formed by polysaccharides (e.g., chitosan, xanthan gum or hyaluronic acid).

## Claims

1. A urinary catheter product including a catheter (14) for insertion into the urethra, wherein the urethra contains urethral mucus having a viscosity, the catheter product comprising:
a package (12) containing:
the catheter (14) comprising a catheter shaft (16) having a hydrophilic coating on the catheter shaft (16);
a hydration medium (18) for hydrating the hydrophilic coating; and
a compound contained in the hydration medium (18), the compound for retaining and/or increasing the viscosity of the urethral mucus and, wherein the compound comprises a desiccant that removes moisture from the urethral mucus.

2. The urinary catheter product of claim 1, wherein the desiccant comprises alcohol.

3. The urinary catheter product of any one of claims 1-2, wherein the desiccant comprises volatile solvents.

4. The urinary catheter product of any one of claims 1-3, wherein the desiccant comprises antihistamines.

5. The urinary catheter product of any one of claims 1-4, wherein the desiccant comprises loperamide hydrochloride.

## Patentansprüche

1. Harnkatheterprodukt, das einen Katheter (14) zum Einführen in die Harnröhre beinhaltet, wobei die Harnröhre Harnröhrenschleim mit einer Viskosität enthält, wobei das Katheterprodukt umfasst:
eine Verpackung (12), die enthält:
den Katheter (14), der einen Katheterschaft (16) umfasst, der eine hydrophile Beschichtung auf dem Katheterschaft (16) aufweist;
ein Hydratationsmedium (18) zum Hydratisieren der hydrophilen Beschichtung; und
eine Verbindung, die in dem Hydratationsmedium (18) enthalten ist, wobei die Verbindung zum Beibehalten und/oder Erhöhen der Viskosität des Harnröhrenschleims dient und wobei die Verbindung ein Trocknungsmittel umfasst, das dem Harnröhrenschleim Feuchtigkeit entzieht.

2. Harnkatheterprodukt nach Anspruch 1, wobei das Trocknungsmittel Alkohol umfasst.

3. Harnkatheterprodukt nach einem der Ansprüche 1-2, wobei das Trocknungsmittel flüchtige Lösungsmittel umfasst.

4. Harnkatheterprodukt nach einem der Ansprüche 1-3, wobei die Säure eine schwache Säure umfasst.

5. Harnkatheterprodukt nach einem der Ansprüche 1-4, wobei das Trocknungsmittel Loperamidhydrochlorid umfasst.

## Revendications

1. Dispositif de cathéter urinaire comportant un cathéter (14) destiné à être inséré dans l'urètre, dans lequel l'urètre contient du mucus urétral ayant une viscosité donnée, le dispositif de cathéter comprenant :
un paquet (12) contenant :
le cathéter (14) comprenant une tige de cathéter (16) ayant un revêtement hydrophile sur la tige de cathéter (16) ;
un milieu d'hydratation (18) pour hydrater le revêtement hydrophile ; et
un composé contenu dans le milieu d'hydratation (18), le composé destiné à retenir et/ou à augmenter la viscosité du mucus urétral et, dans lequel le composé comprend un dessiccant qui élimine l'humidité du mucus urétral.

2. Produit de cathéter urinaire selon la revendication 1, dans lequel le dessiccant comprend de l'alcool.

3. Produit de cathéter urinaire selon l'une quelconque des revendications 1 et 2, dans lequel le dessiccant comprend des solvants volatils.

4. Produit de cathéter urinaire selon l'une quelconque des revendications 1 à 3, dans lequel le dessiccant comprend des antihistaminiques.

5. Produit de cathéter urinaire selon l'une quelconque des revendications 1 à 4, dans lequel le dessiccant comprend du chlorhydrate de lopéramide.
